# FASCICULE DE BREVET EUROPEEN

(11) **EP 0 941 241 B1**
(45) Date de publication et mention de la délivrance du brevet: **06.08.2008**
(21) Numéro de dépôt: 97944948.5
(22) Date de dépôt: 09.10.1997
(51) Int. Cl.: C07K 14/235, C07K 19/00, A61K 38/16

(54) **SOUCHE DE BORDETELLA EXPRIMANT DE LA FHA HYBRIDE**
BORDETELLA-STAMM DIE EINE HYBRIDE-FHA EXPRIMIERT
BORDETELLA STRAIN EXPRESSING THE FHA HYBRID,

(30) Priorité: 11.10.1996 FR 9612461
(43) Date de publication de la demande: 15.09.1999
(73) Titulaire: INSTITUT PASTEUR DE LILLE, 59019 Lille Cédex (FR); INSTITUT NATIONAL DE LA SANTE ET DE LA RECHERCHE MEDICALE (INSERM), 75654 Paris Cédex 13 (FR)
(72) Inventeur: MIELCAREK, Nathalie, F-59000 Lille (FR); LOCHT, Camille, F-59830 Wannehain (FR); RIVEAU, Gilles, F-59800 Lille (FR); POULAIN-GODEFROY, Odile, F-59130 Lambersart (FR); CAPRON, André, F-59113 Phalempin (FR)
(74) Mandataire: Michelet, Alain
(86) Numéro de dépôt international: PCT/FR1997/001802
(87) Numéro de publication internationale: WO 1998/016553

(56) Documents cités:
- EP-A- 0 159 003
- EP-A- 0 322 115
- EP-A- 0 396 964
- EP-A- 0 629 696
- WO-A-87/03301
- WO-A-95/28486
- DATABASE WPI Week 8943 Derwent Publications Ltd., London, GB; AN 89-310022 XP002035139 & DD 268 161 A (STAAT. IMMUNP. NAHRM.) , 24 mai 1989
- DATABASE WPI Week 9135 Derwent Publications Ltd., London, GB; AN 91-260180 XP002035140 , 23 juillet 1991 & US 7 532 327 A (NATIONAL INSTITUTE OF HEALTH) 23 juillet 1991
- G. RENAULD-MONGENIE ET AL.: "Induction of mucosal immune responses against a heterologous antigen fused to filamentous hemagglutinin after intranasal immunization with Bordetella pertussis" PROCEEDINGS OF THE NATIONAL ACADEMY OF SCIENCES, vol. 93, no. 15, 1 juillet 1996, WASHINGTON, DC, US, pages 7944-7949, XP002035136 cité dans la demande
- W. J. BLACK AND S. FALKOW: "Construction and characterization of Bordetella pertussis toxin mutants" INFECTION AND IMMUNITY, vol. 55, no. 10, 1987, WASHINGTON,DC,US, pages 2465-2470, XP002035137
- N. MIELCAREK ET AL.: "Intranasal priming with recombinant Bordetella pertussis for the induction of a systemic immune response against a heterologous antigen" INFECTION AND IMMUNITY, vol. 65, no. 2, 1997, WASHINGTON, DC, US, pages 544-550, XP002035138

## Description

La présente invention est relative à une souche de *Bordetella* pertussis déficiente dans la production de toxine PTX et exprimant une protéine hybride.

Elle est de plus relative à son utilisation immunoprophylactique et/ou thérapeutique, et en particulier à titre de vaccins.

Elle a pour autre objet l'utilisation de la FHA pour la stimulation de la réponse immunitaire.

La coqueluche, dont l'agent majoritairement responsable est la bactérie Gram négative *Bordetella pertussis,* reste une maladie infectieuse d'actualité dans le monde. Plus de 50 millions de cas sont déclarés chaque année et la mortalité est estimée à 500 000 décès par an, principalement chez les nourrissons. La mortalité est notamment élevée dans les pays en voie de développement où la vaccination est insuffisante et où la coqueluche représente une des causes majeures de mortalité infantile. L'introduction de vaccins cellulaires, composés de *B*. *pertussis* inactivées chimiquement ou par la chaleur a entraîné une réduction spectaculaire de l'incidence de la coqueluche. Cependant, des limitations concernant leur utilisation sont apparues au cours de ces deux dernières décennies. Plusieurs rapports ont ainsi émis l'hypothèse d'une relation entre l'administration du vaccin cellulaire contre la coqueluche et certains effets secondaires graves.

De plus, la nécessité d'administrer plusieurs doses vaccinales pour obtenir une protection optimale, la variabilité dans l'efficacité des différents lots de vaccins, et la résurgence de la maladie chez les adultes due à un affaiblissement au cours du temps de l'immunité post-vaccinale en l'absence de rappels tardifs sont autant d'inconvénients qui ont mené au développement de vaccins acellulaires. Néanmoins, les antigènes protecteurs composant ces vaccins de nouvelle génération, généralement qualifiés de plus efficaces, plus sûrs et plus immunogènes que les vaccins cellulaires, n'ont pas été encore clairement définis, et de multiples injections sont toujours nécessaires. De plus, les corrélations sérologiques avec la protection chez les enfants n'ont toujours pas été déterminées. Enfin, le coût de production de ces vaccins se révèle plus élevé que celui des précédents, ce qui représente un problème important pour les pays en voie de développement qui sont les plus demandeurs.

D'autre part, il a été démontré que l'infection naturelle par *B*. *pertussis* entraîne une protection à long terme, alors que celle induite par la vaccination est d'une durée limitée (Bass, J.W. et al. 1987. Pediatr. Infect. Dis. J. 6:141-144; Jenkinson, D. 1988. Br. Med. J. 296:612-614). Les raisons de cette différence ne sont pas claires, ce qui illustre le manque de connaissances concernant les mécanismes immunitaires impliqués dans chaque cas. Une des explications potentielles est basée sur une meilleure induction d'une mémoire immunologique au niveau du tractus respiratoire après une infection, comparée à celle obtenue après une immunisation parentérale. De plus, une réponse cellulaire T de type Th1, qui pourrait jouer un rôle important dans l'immunité protectrice, est induite suite à l'infection naturelle, alors que l'immunisation avec le vaccin acellulaire se traduit plutôt par une forte réponse Th2 (Mills, K.H.G. et al. 1993. J. Med. Microbiol. 39:163-164). Enfin, l'infection stimule la production d'IgA contre les antigènes de *B. pertussis* dans le sérum et dans les sécrétions, alors que ces anticorps sont rarement détectés chez les individus vaccinés (Shahin, R. et al. 1992. In J.E. Ciardi et al. Eds. Genetically Engineered Vaccines. Plenum Pres N.Y.).

L'ensemble de ces observations laisse penser qu'une protection de longue durée contre la coqueluche serait davantage obtenue par une vaccination à l'aide de bactéries vivantes atténuées administrées au niveau des muqueuses respiratoires, portes d'entrées naturelles durant l'infection par *B*. *pertussis.* De précédents travaux ont montré que l'immunisation de souris avec des souches vivantes atténuées, spontanées (Vesselinova-Jenkins, C.K. 1985. Dev. Biol. Stand. 61:517-524) ou aroA (Roberts, F. et al. 1990. Infect. Immun. 58:732-739), de *B. pertussis* protège contre une seconde infection. Cependant, ces souches ont l'inconvénient d'être mal caractérisées et de conserver une certaine toxicité, ou d'avoir perdu en grande partie leur capacité de colonisation et de nécessiter ainsi plusieurs administrations pour induire une immunité protectrice efficace.

La toxine de *B.pertussis* est une protéine oligomérique composée de cinq sous-unités appelées S1 à S5 Elle peut être sous-divisée en deux grands domaines, appelés le protomère A (composée de S1) et l'oligomère B (composé de S2 à S5). L'oligomère B est responsable de l'interaction de la toxine avec ses récepteurs à la surface des cellules cibles et le protomère A ( ou S1) est injecté dans la cellule cible et y exprime une activité enzymatique d'ADP-ribosyltransférase (Tamura et al., Biochemistry 21, 5516-5522, 1982). Les gènes codant les cinq sous-unités ont été clonés et séquencés (Locht et Keith, Science 232, 1258-1264, 1986) et la structure cristalline a été établie (Stein et al., Structure 2, 45-47, 1994). Plusieurs mutations dans le gène de la toxine ont été décrits comme étant responsables de l'inactivation génétique de cette molécule. Ces mutations peuvent se trouver dans le gène de S1 (revue, voir Locht et Antoine. Biochimie 77, 333-340, 1995) et/ou dans les gènes codant l'oligomère B (voir comme exemple Lobet et al. J. Exp. Med. 177, 79-87, 1993). La toxine peut être mise en évidence par réactivité immunologique avec des anticorps polyclonaux ou monoclonaux, tels que par exemple l'anticorps 1B7 mentionné ci-dessous, ou par son activité biologique, tel que l'activité sur les cellules « Chinese Hamster Ovary » comme décrit par Hewlett et al. (Infect. Immun. 40, 1198-1203, 1983).

La FHA est l'adhésine majeure produite et sécrétée par B. *pertussis.* (pour revue voir Locht et al, Mol. Microbiol., 9, 653-660, 1993). Le gène de structure de la FHA, appelé fhaB, a été cloné (Brown, D.R. et al. 1987. Infect. Immun. 55:154-161; Relman, D.A. et al. 1989. Proc. Natl. Acad. Sci. USA. 86:2637-2641; Delisse-Gathoye, A.M. et al. 1990. Infect. Immun. 58:2895-2905) et code pour un précurseur (FHAB) d'environ 367 kDa. La forme mature (220 kDa) correspond aux deux tiers N-terminaux de FHAB et possède une structure dite « en épingle à cheveux » (Makhov et al, J. Mol. Biol., 241, 110-124, 1994). Bien que la partie C-terminale de FHAB ne soit pas présente dans la forme mature, elle possède des caractéristiques intéressantes telles que plusieurs régions réputées riches en proline et un site RGD. De plus, cette partie C-terminale du précurseur semble jouer un rôle important dans la sécrétion de la FHA. En aval de la région N-terminale de la FHA se trouve deux régions répétées, respectivement A et B, suivies par un site sensible à la protéolyse et par une séquence RGD. Le site de maturation de FHAB est situé à environ 1000 acides aminés en aval de cette séquence RGD. Le domaine N-terminal de FHAB joue un rôle essentiel dans la sécrétion de la protéine mature puisqu'une délétion en phase de cette région semble totalement inhiber la biogenèse de la FHA. La sécrétion de la FHA dépend d'une protéine accessoire dénommée FhaC, présente au niveau de la membrane externe de *B. pertussis.* Cette protéine est codée par le gène FhaC localisé en aval de FhaB et séparé de ce dernier par trois autres gènes, fimB, fimC et fimD, codant pour les fimbriae. Un mécanisme de sécrétion de la FHA impliquant un clivage puis une modification de la partie N-terminale de FHAB, suivie d'une interaction entre cette région et la protéine accessoire FHAC a été proposé.

La variété des mécanismes de fixation de la FHA permet l'adhérence de *B. pertussis à* des nombreux types de cellules eucaryotes. Bien que la toxine de *B. perfussis* soit impliquée dans l'adhérence de la bactérie aux cellules épithéliales du tractus respiratoire, la FHA joue un rôle prépondérant dans cette interaction. La FHA peut interagir avec des glycoconjugués et le domaine de reconnaissance des carbohydrates a été identifié dans la région de la FHA délimitée par les acides aminés 1141 à 1279, qui correspondait à la boucle de la structure « en épingle à cheveux » qui contient déjà le site RGD en position 1097-1099. La FHA est également l'adhésine majeure impliquée dans le mécanisme d'adhérence de *B. pertussis* aux cellules épithéliales non ciliées. Une activité de fixation de cette molécule sur l'héparine, glycosaminoglycane, sulfaté présent en quantité non négligeable dans le mucus bronchique ainsi que dans la matrice extracellulaire et à la surface de nombreuses cellules épithéliales, est responsable de l'adhérence de la bactérie sur ces cellules. Des études récentes mettent en évidence un site de fixation à l'héparine spécifique localisé dans la région N-terminale de la FHA, probablement dans la région comprise entre les acides aminés 442 et 863 (Hannah et al., Infect. Immun, 62, 5010-5019, 1994).

En plus de cette activité d'adhérence sur les cellules épithéliales ciliées et non-ciliées, la FHA est capable de se lier spécifiquement au CR3 (CD11b/CD18, aMb2) présent sur les macrophages et les monocytes (Relman et al, Cell, 61, 1375-1382, 1990). Cette fixation de *B. pertussis,* via la FHA, au CR3 permet l'internalisation des bactéries dans les macrophages par une voie n'engendrant pas de catabolisme oxydatif.

De par son importance dans l'adhérence de *B.pertussis* à différents types cellulaires, la FHA a été considérée comme un antigène majeur pour la vaccination contre la coqueluche. L'administration d'un vaccin cellulaire à des souris ou à des enfants entraîne la production d'anticorps anti-FHA dans leur sérum. De plus, la vaccination d'enfants âgés de 18 mois avec un vaccin acellulaire induit des taux élevés d'anticorps anti-FHA ainsi qu'une réponse cellulaire T spécifique. Ces caractéristiques immunes sont retrouvées chez les personnes convalescentes après une coqueluche.

Cependant, le profil isotopique de la réponse anti-FHA chez des enfants ayant reçu le vaccin cellulaire diffère de celui des individus atteints par la coqueluche. Alors que le taux d'immunoglobulines G (IgG) sérique spécifique est similaire, la réponse IgA2 anti-FHA est plus importante chez les malades que chez les personnes vaccinées. Cette différence est encore plus marquée au niveau des sécrétions nasopharyngées et de la salive où l'on observe une augmentation du taux d'IgA anti-FHA chez la plupart des malades plusieurs semaines après le début des symptômes. De manière similaire, on retrouve des taux élevés d'IgA anti-FHA dans les sécrétions nasales de souris infectées avec la souche virulente de *B. pertussis* et ce, sur une période allant jusqu'à 26 semaines après l'infection. Une réponse immune, à la fois IgAs et IgG, contre la FHA peut également être obtenue dans les voies respiratoires de la souris après vaccination intranasale avec de la FHA purifiée.

Des épitopes de cellules B et T potentiellement protecteurs ont été localisés sur la FHA. Une région immunodominante a ainsi été déterminée dans la région C-terminale de la FHA mature. Elle est reconnue par les cellules B murines et par les lymphocytes T CD4+ humains qui reconnaissent également la partie N-terminale de la FHA.

Des protéines de fusion recombinantes comprenant une partie de la FHA et des peptides hétérologues ont été décrits dans la demande FR 94 04 661 (N° de publication 2.718.750) (INSTITUT PASTEUR, INSTITUT PASTEUR DE LILLE, INSERM). Dans cette demande, des ADN recombinants contenant une séquence codant pour un polypeptide hétérologue, fusionnée dans le même cadre de lecture a une séquence codant pour une partie au moins du précurseur de la FHA, sont préparés et exprimés dans des cultures cellulaires, en particulier des cultures de *Bordetella* détoxifiées ou atténuées.

L'un des objectifs de cette demande est l'exposition du peptide recombinant à la surface de cellules procaryotes, notamment à des fins de vaccination. On notera que la détoxification, ou l'atténuation, de cellules recombinantes n'est mentionnée qu'à titre accessoire, et que par ailleurs aucun exemple ou aucun mode de mise en oeuvre particulier ne vient illustrer cette éventualité. En particulier, cette demande ne précise pas de quelle manière les cellules sont détoxifiées ou atténuées.

Il ressort donc de l'analyse de l'état de la technique que l'on ne connaissait des moyens de vaccination contre la coqueluche, mais qu'ils induisaient des effets secondaires.

Le demandeur s'est donc attaché à trouver des moyens de vaccination contre la coqueluche, mais aussi contre d'autres agents pathogènes, dépourvus d'effets secondaires, faciles à mettre en oeuvre et pouvant être produits à faible coût.

Il a tout d'abord montré de manière surprenante que l'administration intranasale d'une souche dépourvue du gène de la toxine de *B. pertussis* induit une production élevée d'anticorps dirigés contre l'hémagglutinine filamenteuse (FHA), et qu'il est ainsi possible de stimuler la réponse immunitaire contre un antigène hétérologue fusionné à la FHA.

Il a d'autre part montré que la FHA, intégrée dans des liposomes contenant d'autres peptides présentant des épitopes, permet d'améliorer la fixation de ces liposomes sur les muqueuses des voies respiratoires.

Il a enfin montré que la FHA présentait de manière générale des propriétés immunostimulantes.

La présente invention est donc tout d'abord relative à une souche de Bordetella pertussis déficiente dans la production de toxine PTX, par élimination, délétion partielle ou mutation du gène de la toxine PTX et exprimant et exposant à sa surface une protéine hybride comprenant au moins une partie de l'hémagglutinine filamenteuse (FHA) et au moins une partie d'une protéine hétérologue vis-à-vis de la FHA.

De manière avantageuse, la protéine hétérologue constituant une partie de la protéine hybride, comprend au moins un épitope d'une protéine susceptible d'être exprimée par des agents pathogènes lors d'affections des voies respiratoires supérieures ou inférieures.

Ainsi, ladite protéine hybride peut comprendre une partie de la protéine FHA et une partie de la protéine Sm28GST de Schistosoma mansoni. Cette protéine particulière peut être exprimée dans une souche de l'espèce *B*. *pertussis,* et être en particulier la souche BPNX déposée sous le n°I-1770 le 8 Octobre 1996 auprès de la.Collection Nationale de Cultures de Microorganismes de l'Institut Pasteur (CNCM).

Une souche selon la présente invention peut être obtenue par élimination du gène de la toxine du génome d'une souche virulente exprimant ladite protéine hybride, ou par délétion partielle ou par mutation afin de produire une toxine inactive.

L'élimination peut être effectuée par toute méthode connue de l'homme du métier, et en particulier par croisement de la souche virulente avec une souche mobilisante, puis par sélection, à l'aide de marqueurs adaptés en fonction des souches des cellules ayant perdu le gène de la une telle perte de la capacité de la souche virulente à exprimer la toxine résulte d'un double événement de recombinaison homologue entre la souche virulente et un plasmide de la souche mobilisante. L'homme du métier peut se référer pour l'obtention de souches atténuées à la méthode décrite par Antoine et Locht (1990, Infect. Immun, 58, 1518-1526).

Les caractéristiques des souches déficientes dans la production de toxine, ainsi sélectionnées, peuvent être vérifiées par différentes techniques, en particulier par Western-blot.

Les souches virulentes exprimant la protéine hybride peuvent être obtenues par les techniques connues de l'homme du métier, et en particulier être obtenues comme décrit dans la demande FR-94. 04 661 mentionnée ci-dessus. Les ADN recombinants comprenant d'une part une séquence codant pour un peptide hétérologue et d'autre part une séquence pour une partie de la FHA sont obtenus par les méthodes connues de l'homme du métier, et en particulier comme décrit dans l'exemple V de la demande FR 94 04 661. Cet exemple aboutit à la fusion de la région 190-211 de la glutathion-S-transférase de 28 kDa (Sm28GST) de *Schistosoma mansoni*, avec la protéine FHA tronquée. Les ADN recombinants codant pour les protéines hybrides sont sélectionnés, séquence est vérifiée, selon des méthodes connues de l'homme du métier, puis ils sont transférés dans des cellules de *Bordetella.*

L'homme du métier pourra éventuellement se référer, pour la mise en oeuvre de la présente invention, à des manuels généraux ayant trait à ces techhiques, et en particulier au manuel suivant : Maniatis et al., 1982, Molecular Cloning: Laboratory Manual, Cold Spring Harbor Laboratory, Cold Spring Harbor New York, USA, ou à l'une de ses récentes rééditions.

La protéine hétérologue, dont la séquence est comprise dans la protéine hybride, peut être toute séquence protéine antigénique, en particulier des antigènes de *Bordetella,* de *Shigella,* de *Neisseria,* de *Borrellia,* des toxines ou toxoïdes diphtériques, tétaniques ou colériques, des antigènes viraux, notamment de l'hépatite B , de l'hépatite C , du polyovirus ou de HIV, ou des antigènes parasitaires tels que ceux des Plasmodium, des *Schistosoma* ou des toxoplasmes. Elle peut aussi comprendre un épitope d'une protéine susceptible d'être exprimée par des pathogènes lors d'infections mucosales ou lors d'infections systémiques.

De telles souches peuvent être utilisées pour la vaccination de l'être humain ou d'animaux à l'encontre de divers agents pathogènes. Elles peuvent être en particulier administrées par voie intranasale.

La présente invention est donc en outre relative à des médicaments et à des vaccins contenant de telles souches, ou à des compositions pharmaceutiques contenant une quantité pharmacologiquement efficace de telles souches, et éventuellement un ou plusieurs excipients pharmacologiquement compatibles.

Un autre objet de la présente invention consiste en l'utilisation de telles souches pour la fabrication d'un médicament ou d'un vaccin pour le traitement des affections des voies respiratoires inférieures ou supérieures des infections mucosales ou des infections systémiques.

On notera que les souches selon la présente invention présentent des activités surprenantes. En effet, comme le montrent les exemples qui suivent, les souches exprimant des protéines hybrides et dont le gène de la toxine a été éliminé, induisent une production d'anticorps bien plus élevée que celle obtenue avec la souche parentale virulente, lorsque ces deux souches sont administrées par voie intranasale. L'administration intranasale, par exemple par vaporisation nasale, permet d'éliminer les risques de contamination liés à une injection à travers la peau et évite également la destruction des vaccins administrés par voie orale dans l'environnement acide de l'estomac.

La présente demande décrit aussi des liposomes comprenant au moins une partie de la protéine FHA, ainsi qu'au moins une protéine hétérologue à la protéine FHA.

Une telle protéine hétérologue peut être celle composant la protéine hybride décrite ci-dessus. Elle peut ainsi comprendre au moins un épitope d'une protéine susceptible d'être exprimée lors d'affections des voies respiratoires supérieures ou inférieures. De tels liposomes peuvent être obtenus d'une manière connue par l'homme du métier, par un procédé comprenant le mélange de la FHA, de la protéine hétérologue et des lipides, puis la réhydratation du film lipidique. Ils peuvent être utilisés à des fins thérapeutiques pour la fabrication de médicaments et de vaccins pour la stimulation de la réponse immunitaire à l'encontre d'un épitope donné.

La présente demande concerne donc des médicaments contenant de tels liposomes, ainsi que des compositions pharmaceutiques contenant des quantités pharmacologiquement efficaces de tels liposomes, ainsi qu'éventuellement des excipients pharmaceutiquement compatibles.

La fixation de la FHA à la surface des liposomes présente l'avantage de cibler vers le tissu muqueux l'administration de la protéine hétérologue. La FHA va présenter ainsi deux actions: d'une part une action de ciblage des liposomes, due à une propriété d'adhérence particulière vis-à-vis des tissus muqueux , et d'autre part des propriétés immunostimulantes qui vont permettre d'augmenter la réponse immune à l'encontre des antigènes de la protéine hétérologue.

La présente invention est illustrée sans pour autant être limitée par les exemples qui suivent.

La figure 1A illustre la colonisation des poumons de souris OF1 par les souches de *B. pertussis* BPSM (PTX⁺, FHA⁺), BPRA (PTX⁻, FHA⁺), et BP347 (PTX⁻. FHA⁻, PRN⁻, Fim⁻, AcHly⁻).

La figure 1B illustre quant à elle la colonisation des poumons par des bactéries BPSM de souris, préalablement immunisées intranasalement par les souches de la figure 1A ou par *S. typhimurium.* Trois heures après l'infection, 3 souris par groupe ont été sacrifiées et le nombre de *B. pertussis* viables estimé par poumon. Les autres groupes de souris ont été analysés une ou plusieurs semaines après infection comme montré sur la figure. Les barres représentent les déviations standard à la moyenne (SEM).

La figure 2A représente des poumons de souris OF1 saines (1) et 7 jours après administration nasale de 5 x 10⁶ bactéries des souches BPSM (2), BPRA (3).

Les figures 2B à 2D sont des coupes de poumons 14 jours après administration intranasale de 5 x 10⁶ bactéries des souches BPSM (2C), BPRA (2D); le témoin correspond à des souris saines (2B). La coloration des noyaux cellulaires est obtenue par traitement à l'hématoxyline.

La figure 3 illustre la réponse IgG anti-FHA 28 jours après administration intranasale de souris OF1 par les souches BPSM, BPRA, et BP347, et 28 jours après la seconde infection par BPSM (jour 63). L'évaluation du taux d'anticorps a été déterminée par ELISA pour trois souris par groupe et par temps.

Les figures 4A à 4D représentent les cinétiques de la distribution isotypique anti-FHA. Les sérums de trois souris par temps ont été analysés pour la présence d'anticorps anti-FHA d'isotype IgG1 (4A), IgG2a (4B), IgG2b (4C), et IgA (4D) par technique ELISA.

Les figures 5A et 5B représentent les cinétiques de la réponse sérique IgG2a contre les antigènes de *B. pertussis.* Les sérums de trois souris par temps ont été analysés pour la présence d'anticorps anti-BP347 (5A) et anti-BPSM (5B) par technique ELISA.

La figure 6 représente la cinétique de la réponse sérique IgG anti-FHA après administration des souches de *B.pertussis* BPSM (PTX⁺), BPRA (PTX⁻), et BPDS1 (S1-; oligomère B+). Les taux d'anticorps ont été déterminés par ELISA pour trois souris par groupe et par temps.

La figure 7 illustre l'analyse par Western-blot des surnageants de culture des souches non-recombinantes BPSM (PTX⁺) (puits 4) et BPRA (PTX⁻) (puits 1) et des souches recombinantes exprimant la protéine FHA-Sm28GST BPGR60 (PTX⁺) (puits 3) et BPNX (PTX⁻) (puits 2) en utilisant l'anticorps monoclonal 1B7 dirigé contre la sous-unité S1 de PTX. Le puits 5 correspond au marqueur de poids moléculaire.

La figure 8 représente la cinétique de la réponse sérique IgG anti-Sm28GST après administration intranasale à des souris OF1 de souches recombinantes de *B. pertussis* BPGR60 (PTX⁺) et BPNX (PTX⁻).

La figure 9 est un immunotransfert (immunoblot) de différentes préparations liposomales (Sm28GST seule puits 6 et 13, Sm28GST avec trois doses différentes de FHA 60 µg/lml (puits 3 et 10), 6 µg/ml (puits 4 et 11) et 0,6 µg/ml (puits 5 et 12) analysées après transfert d'un gel SDS-PAGE sur nitrocellulose. Les puits 1 et 8 correspondent à la Sm28GST non incluse dans des liposomes. Les puits 2 et 9 correspondent à la FHA non incluse dans des liposomes. Le puits 7 est celui du marqueur de poids moléculaire.

Les puits 1 à 6 ont été révélés avec un anticorps spécifique pour la FHA et les puits 8 à 13 avec un anticorps spécifique pour la Sm28GST (190-211). La Sm28GST dimérisée migre différemment en présence de phospholipides. La présence de FHA n'a pas modifié la quantité de Sm28GST incorporée aux liposomes.

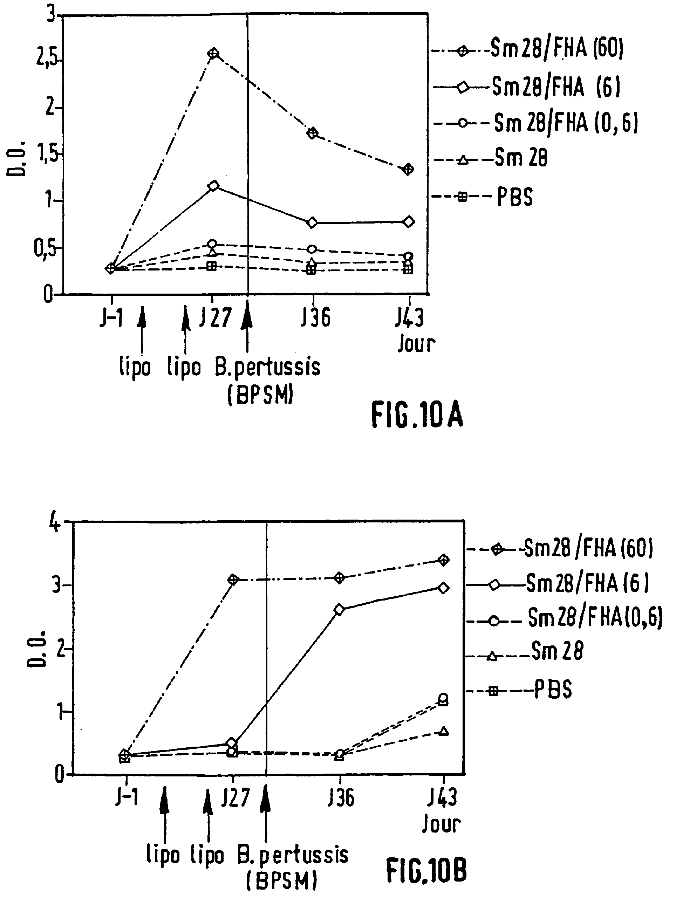

Les figures 10A et 10B illustrent respectivement les réponses sériques IgG(H+L) anti-Sm28GST et anti-FHA (pool sérum 1/40 révélation 1 heure en ABTS). Deux instillations par voie nasale à J1 et J14 ont été pratiquées. Les sérums ont été analysés à J-1 et J27, et à J36 et J43 après une infection par *B. pertussis* qui a eu lieu à J28.

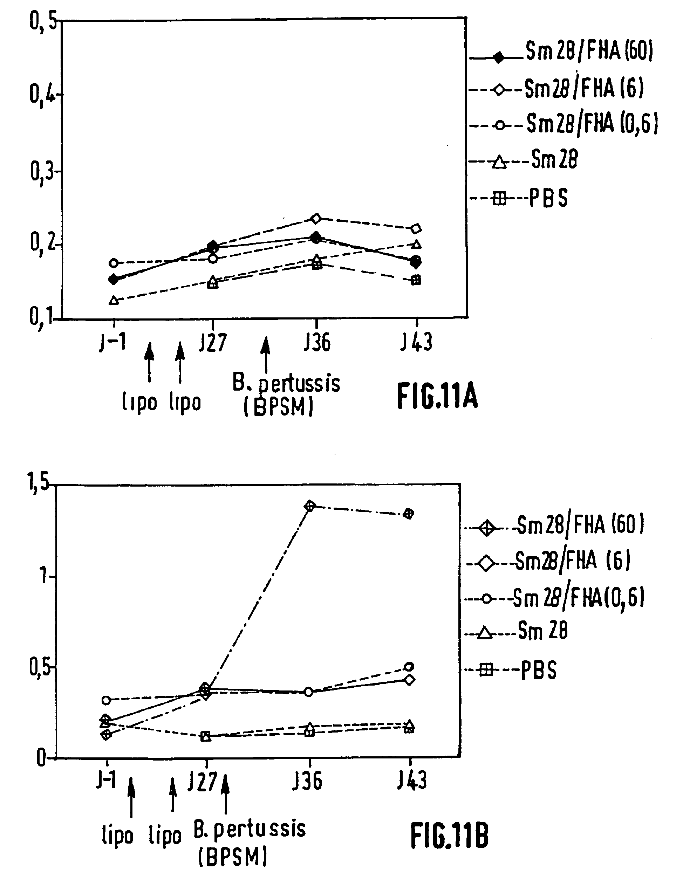

Les figures 11A et 11B illustrent respectivement les réponses sériques IgA anti-Sm28GST et anti-FHA (pool sérum 1/40 révélation en OPD). Deux instillations par voie nasale à J1 et J14. Les sérums ont été analysés à J-1 et J27, et à J36 et J43 après une infection par *B*. *pertussis* qui a eu lieu à J28.

### EXEMPLE 1:

### Protection contre une infection par B. pertussis préalablement immunisée avec différentes souches déficientes dans la production d'un ou de plusieurs facteurs de virulence.

La souche sauvage de *B. pertussis* BPSM (Menozzi, F.D. et al. 1994, Infect. Immunol., 62, 769-778) a été utilisée comme référence pour la protection contre une seconde infection avec BPSM. L'efficacité de la souche BPRA (Antoine R. et al. 1990. Infect. Immun. 58:1518-1526), déficiente dans la production de la toxine de *pertussis* (PTX) a été comparée à la souche BP347, possédant une insertion du transposon Tn5 dans son génome entraînant une incapacité d'exprimer les facteurs de virulence tels que la PTX, la toxine adénylate-cyclase (Ac-Hly), les fimbriae, la pertactine et la FHA (Weiss, A.A. et al. 1983. Infect. Immun. 42:33-41). Une souche de *Salmonella typhimurium* aroA (Hoiset, S.K. et al. 1981. Nature 291:238-241) a été administrée par voie intranasale à un groupe de souris comme témoin d'infection par des bactéries Gram négatives. 5 x 10⁶ bactéries *B. pertussis* en suspension dans du PBS stérile (cellules grattées d'une culture sur milieu solide, Bordet Gengou avec du sang défibriné de mouton (BG); Bordet, J. et Gengou, O. 1906. Ann. Institut Pasteur (Paris). 20:731-741) ont été instillées nasalement à raison de 25 µl par narine, à des souris OF1 (souche non pure, souris femelles âgées de 4 semaines) sous anesthésie au pentobarbital. Les bactéries *S. typhimurium* provenant d'une culture sur la nuit à 37°C en milieu liquide LB ont été centrifugées pendant 10 mn à 6000 trs/mn, et le culot a été resuspendu dans du PBS stérile à une concentration de 2 x 10³ bactéries/ml. Les souris ont alors été instillées avec 50 µl de la solution bactérienne. Les poumons de 3 souris ont été prélevés 3 heures après l'injection, puis à 7, 14, 21, 28 et 35 jours après injection. Les poumons ont alors été homogénéisés dans 5 ml de PBS stérile, puis les bactéries comptées 3 à 4 jours après étalement des broyats sur milieu solide BG contenant 100 µg/ml de streptomycine et 25 µg/ml d'acide nalidixique (BGSN). Pour simplifier, le terme « souris X » pour « souris immunisées par X » a été utilisé.

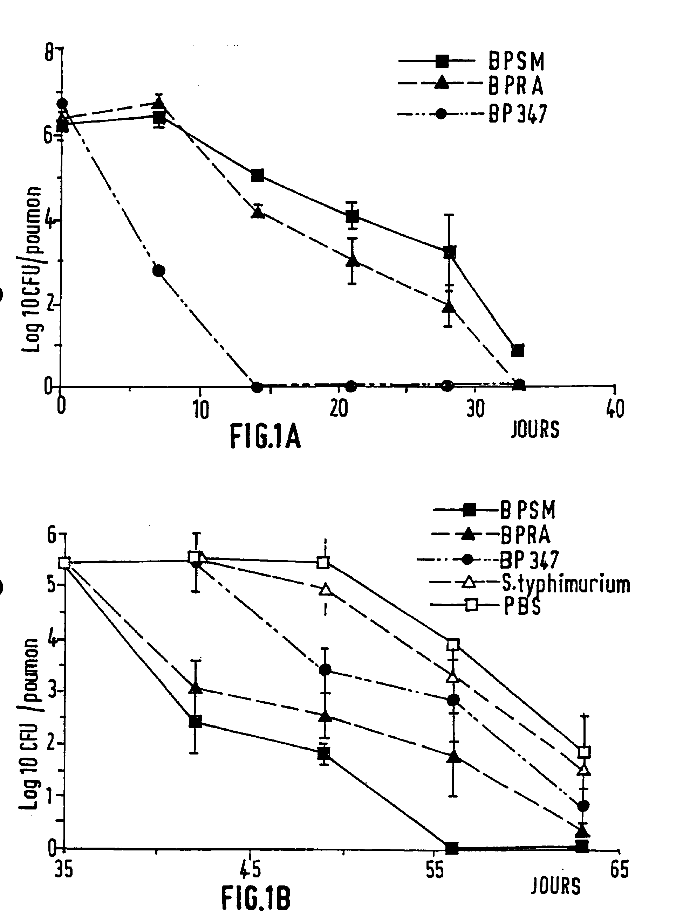

Dans un premier temps, la capacité des différentes souches atténuées de *B. pertussis à* coloniser le tractus respiratoire des souris a été vérifiée. Comme le montre la figure 1A, le nombre des bactéries BPRA augmente dans les poumons des souris durant les sept premiers jours suivant l'instillation, puis chute au cours des quatre semaines suivantes, de la même manière que la souche sauvage BPSM, mais leur cinétique d'élimination est plus rapide. La souche BP347, déficiente dans la production des facteurs de virulence, est incapable de se multiplier durant la première semaine, et 14 jours après l'immunisation, aucune bactérie n'est retrouvée dans les poumons. Enfin, S. *typhimurium,* microorganisme colonisant le tractus gastro-intestinal après une infection par voie orale, est rapidement éliminé car incapable de coloniser les muqueuses des voies respiratoires. Un groupe de souris témoin a reçu intranasalement 50 µl de PBS stérile.

Une seconde infection intranasale avec la souche sauvage (5 x 10⁶ bactéries BPSM/50 µl PBS) est réalisée 35 jours après l'immunisation nasale des souris avec les différentes souches bactériennes. Les souris témoins PBS et les souris S. *typhimurium* montrent une courbe de colonisation similaire (figure 1 B). Chez les animaux BPSM et BPRA, le nombre de bactéries diminue rapidement après la seconde infection par BPSM. 7 jours plus tard, la quantité de bactéries présentes dans les poumons des souris BPRA est 300 fois plus faible que chez les souris PBS, et est semblable à celle observée chez les souris BPSM. Par contre, durant la première semaine suivant l'infection, les bactéries BPSM semblent coloniser les poumons des souris BP347 de la même manière que les poumons des souris PBS. Néanmoins, une semaine plus tard, les animaux BP347 présentent une réduction du nombre de bactéries de l'ordre de 100 fois comparée au groupe PBS. 21 jours après la seconde infection, aucune bactérie n'est détectée dans les poumons des souris BPSM, alors qu'une diminution de l'ordre de 130 fois pour les souris BPRA et de 12 fois pour les souris BP347 comparée aux souris PBS est observée. 4 semaines après l'infection, l'élimination quasi totale des bactéries est atteinte chez les souris BPRA, alors qu'il en reste encore chez les souris PB347.

Ces résultats indiquent que seule la souche BPRA, déficiente dans la production de PTX, se comporte *in vivo* de façon similaire à la souche sauvage et qu'une primo infection par l'une de deux souches protège contre une colonisation efficace des muqueuses respiratoires par la souche sauvage.

### EXEMPLE 2:

### Analyse histologique de l'inflammation au niveau des poumons induite par l'administration intranasale d'une souche de B. pertussis déficiente dans la production de la toxine de pertussis.

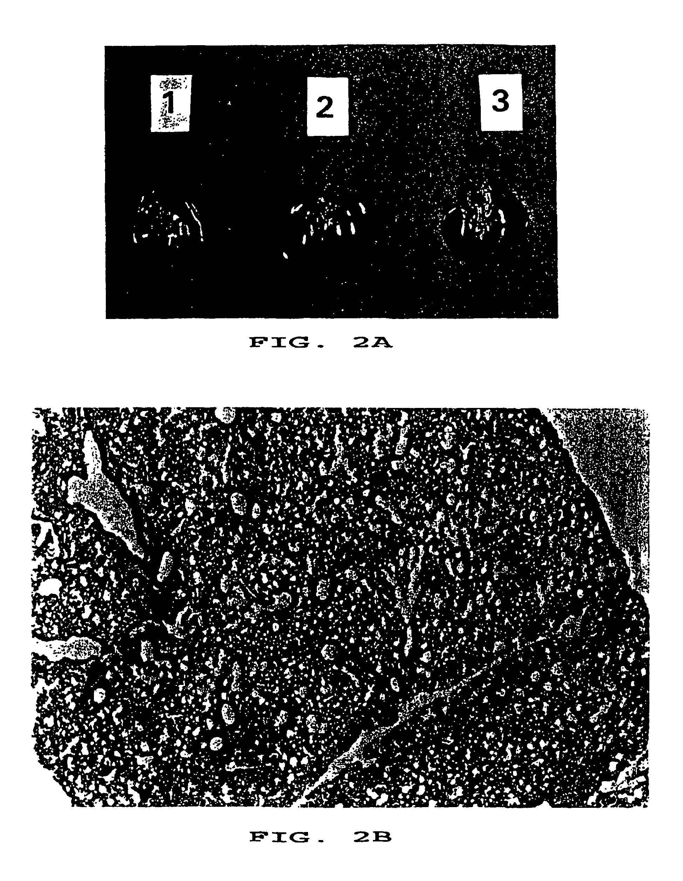

Les souris sont immunisées avec 5 x 10⁶ bactéries (même protocole d'immunisation que celui décrit dans l'exemple 1) provenant de la souche sauvage BPSM ou de la souche BPRA (PTX-). Le nombre de bactéries administrées est vérifié par prélèvement des poumons 3 heures après administration intranasale et par comptage des bactéries après étalement des broyats pulmonaires. 7 jours après administration, l'observation directe montre des poumons de souris BPSM plus volumineux et hémorragiques comparés à ceux des souris BPRA qui ne présentent que quelques petites régions hémorragiques et ont un volume identique à ceux des souris contrôles (figure 2a).

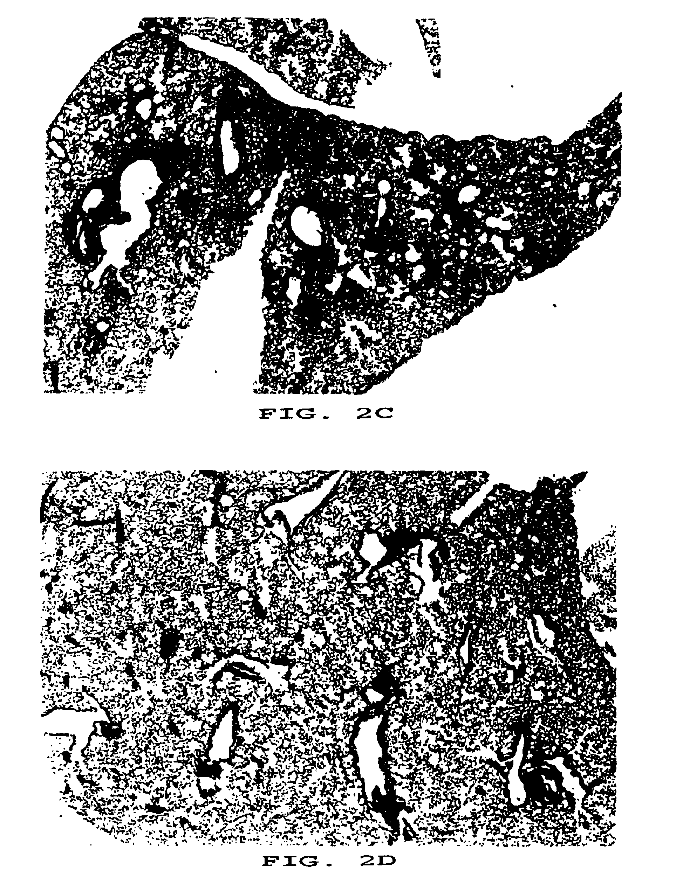

Pour analyser l'intensité de l'infiltration cellulaire, les poumons sont prélevés 14 jours après l'administration de l'une ou l'autre des souches de *B. pertussis.* Ils sont fixés dès le prélèvement dans du paraformaldéhyde à 4%, pendant une nuit à 4°C. Après lavage, les organes sont déshydratés par bains successifs d'éthanol (Merck) à concentrations croissantes, puis mis en contact avec du 1-butanol (Merck) pendant 24h à 4°C pour améliorer l'infiltration de la paraffine. L'inclusion est alors réalisée par infiltration de la paraffine (Paraplast Lancer) 4 à 5h à 56°C. Les coupes sur lames de 6 µm d'épaisseur, sont réalisées au microtome. Préalablement au marquage, les coupes sont déparaffinées au toluène (Merck) et réhydratées. La réhydratation des coupes, réalisées par des bains d'alcool en concentrations décroissantes, se termine pour un bain de tampon TBS (Tris HCl 10 mM, NaCl 150 mM, pH 7,4). La coloration des noyaux cellulaires est effectuée par contact durant 10 mn avec de l'hématoxyline, suivi d'un rinçage des lames pendant 40 mn sous l'eau courante. Les photographies des coupes sont réalisées avec un objectif x10. On observe une infiltration cellulaire au niveau des poumons à la fois chez les souris BPSM et BPRA, les poumons d'une souris saine étant utilisés comme témoin négatif (figure 2b). Cependant, alors que l'infiltration cellulaire est légère et limitée à la périphérie des voies supérieures pour les souris BPRA (figure 2d), elle apparaît massive et a gagné jusqu'aux alvéoles des poumons pour les souris BPSM (figure 2c).

Ces observations indiquent que la souche BPRA, déficiente dans la production de PTX, induit une réaction inflammatoire au niveau pulmonaire beaucoup plus faible que celle provoquée par l'administration nasale de la souche sauvage BPSM.

### EXEMPLE 3:

### Analyse de la réponse immune sérique anti-FHA auprès administration intranasale des différentes souches bactériennes.

Les titres de la réponse IgG totale anti-FHA sont déterminés dans le sérum des souris, 28 jours après administration intranasale des souches de *B. pertussis* (BPSM, BPRA, BP347) ainsi qu'un mois après une seconde infection avec BPSM (même protocole que celui décrit dans l'exemple 1. Les souris sont saignées par ponction au niveau du plexus rétroorbital et les sérums sont conservés à -20°C jusqu'à leur analyse par technique ELISA.

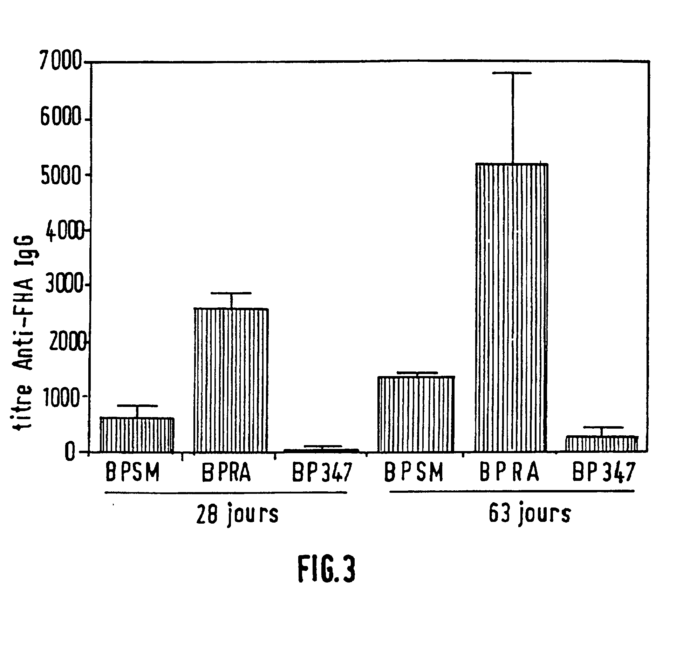

Durant l'intervalle de temps 28-63 jours, les titres de la réponse IgG anti-FHA ont doublé dans chaque groupe de souris immunisées par les différentes souches de *B. pertussis* (figure 3). De manière intéressante, les souris BPRA présentent une réponse anti-FHA 4 fois supérieure à celle observée chez les souris BPSM, différence observée 28 jours après immunisation ou après la seconde infection par BPSM. Ces résultats sont retrouvés indistinctement lorsque les tests ELISA sont effectués contre de la FHA purifiée à partir de la souche BPSM ou BPRA.

Afin de déterminer si les différences dans la réponse sérique anti-FHA des souris BPSM et BPRA étaient uniquement quantitatives et/ou qualitatives, le profil de la réponse anti-FHA a été réalisé par ELISA.

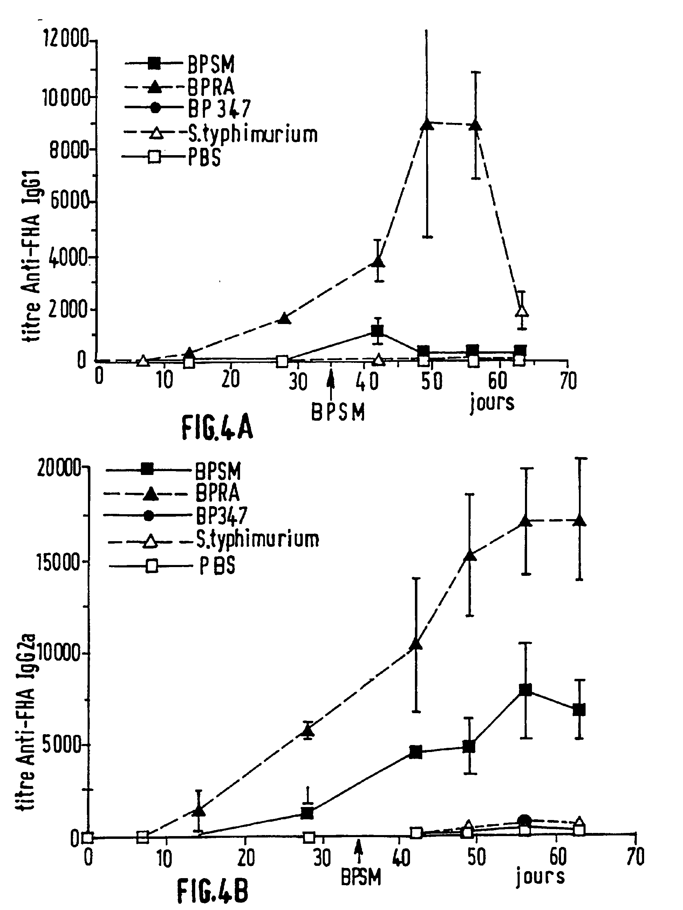

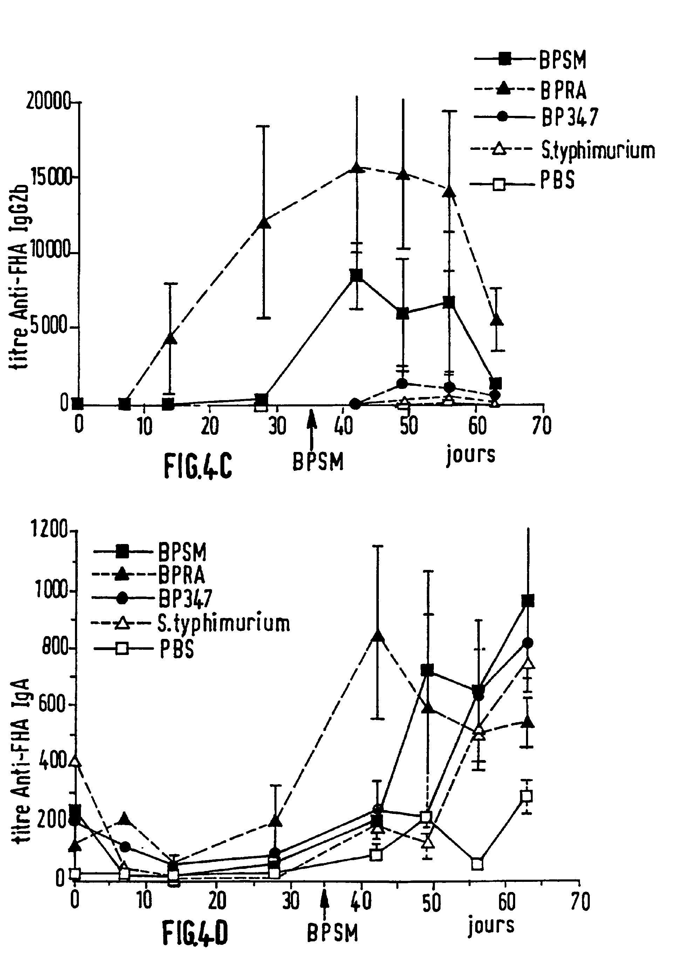

L'analyse des cinétiques de réponse spécifique montre, 14 jours après administration intranasale, la présence d'IgG1 (figure 4a), d'IgG2a (figure 4b) et d'IgG2b (figure 4c) dans le sérum des souris BPRA, alors que des IgG2a et des IgG2b sont détectées chez les souris BPSM uniquement un mois après immunisation. Dans ces deux groupes de souris, la seconde infection par BPSM a induit une augmentation de la réponse IgG2a et IgG2b anti-FHA. D'autre part, alors qu'une forte augmentation de la réponse IgG1 spécifique est observée chez les souris BPRA, une production faible et transitoire est détectée chez les souris BPSM une semaine après la seconde infection. Un mois après cette infection, une diminution du taux d'IgG1 et d'IgG2b est détectée dans le sérum des souris BPRA alors que le taux d'IgG2a reste stable. L'analyse de la réponse IgA sérique anti-FHA montre, une semaine après la seconde infection par BPSM, une production élevée d'IgA spécifique dans le sérum des souris BPSM (figure 4d). Une semaine plus tard, ce niveau d'IgA sérique est atteint chez les souris BPRA, suivi 7 jours plus tard par la détection d'IgA anti-FHA dans le sérum des souris BP347 et des souris S. *typhimurium.*

Ces cinétiques de distribution de la réponse isotypique anti-FHA mettent donc en évidence une réponse sérique spécifique plus précoce et plus élevée dans le sérum des souris BPRA (PTX-) comparée à celle retrouvée dans les souris BPSM (PTX⁺).

Une des hypothèses qui pourrait expliquer ces résultats serait que la quantité de FHA exprimée à la surface et/ou sécrétée par la souche BPRA soit supérieure à celle produite par la souche sauvage BPSM. Une analyse par immunoblot à l'aide d'un anticorps polyclonal de rat anti-FHA, a été réalisée sur des surnageants de culture et des lysats cellulaires provenant d'une culture liquide des différentes souches de B. pertussis en milieu Stainer-Scholte (Stainer, D.W., and Scholte, M.J. 1971. J. Gen. Microbiol. 63:211-220) contenant 1g/l de 2,6-O-diméthyl-β-cyclodextrine (Imaizumi, A. et al. 1983. Infect. Immun. 41:1138-1143). Cette étude a mis en évidence des quantités similaires de FHA produites par la souche BPRA et BPSEM.

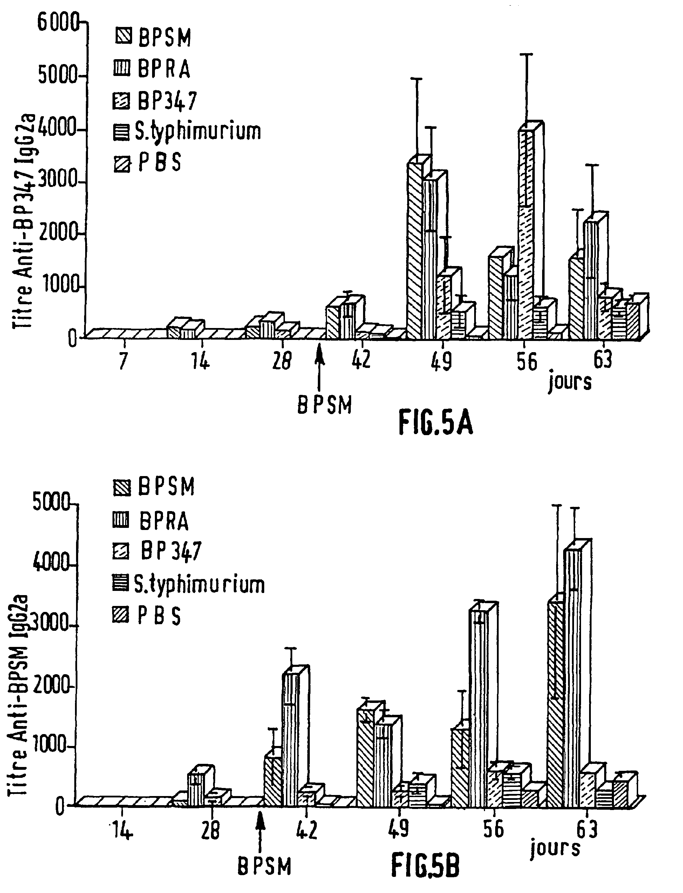

Une activité potentiellement immuno-régulatrice de la PTX, qui agirait comme un élément immunosuppresseur, pourrait être à l'origine de la faible intensité de la réponse anti-FHA chez les souris BPSM (PTX+) comparée à celle obtenue chez les souris BPRA (PTX-). Une étude de la réponse IgG2a, plus sensible que la réponse IgG totale, contre les antigènes totaux des bactéries BP347 et BPSM a été réalisée sur les sérums de chaque groupe de souris. Pour préparer les antigènes totaux, les bactéries ont été striées sur des boîtes BGS, puis après 48h de culture, remises en suspension à une concentration de 2,5 x 10⁹ bactéries/ml dans du PBS contenant 0,5 mM de PMSF et 1 mM d'EDTA. La suspension cellulaire est ensuite lysée par deux passages successifs dans une presse de French. Les taux d'anticorps dirigés contre les antigènes de la souche BP347 (anticorps anti-BP347) sont similaires dans les sérums des souris BPSM et BPRA (figure 5a), alors que de légères différences sont notées quant aux niveaux de la réponse anti-BPSM avec une réponse plus élevée chez les souris BPRA, probablement liée au taux d'IgG2a anti-FHA (figure 5b). De plus, alors que le niveau des anticorps anti-BP347 décroît progressivement dans le sérum des souris BPSM et BPRA, la réponse IgG2a anti-BPSM continue à augmenter au moins jusqu'à 28 jours après la seconde infection par BPSM, et ce de manière parallèle à la réponse IgG2a anti-FHA.

### EXEMPLE 4:

### Etude du rôle de l'activité enzymatique de PTX sur la réponse sérique anti-FHA.

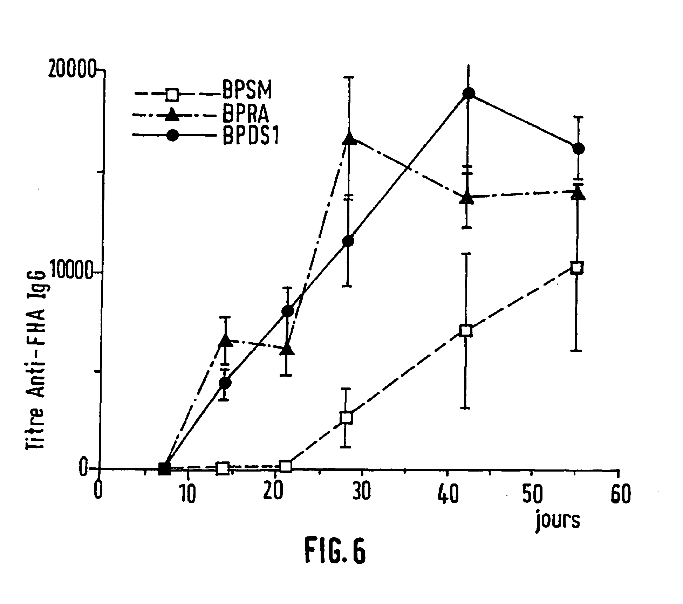

La PTX est une protéine oligomérique composée de 5 sous-unités, appelées S1 à S5 Cette holotoxine possède une structure de type A-B où A correspond à la sous-unité S1 exprimant l'activité enzymatique ADP-ribosyltransférase et NAD glycohydrolase (Katada. T. et al.. 1983. Arch. Biochem. Biophys. 224:290-298). Cette protéine possède 2 cystéines qui forment un pont disulfure quand la toxine est sécrétée par la bactérie. Une de ces cystéines (cys-41) est située près du site de fixation du NAD sur l'enzyme (Locht, C. et al. 1990. J. Biol. Chem. 265:4552-4559) et est essentielle à la stabilité de la sous-unité S1 chez *B. pertussis.* Afin de déterminer le rôle de l'activité enzymatique de PTX sur la réponse anti-FHA, un groupe de souris à reçu 5 x 10⁶ bactéries d'une souche de *B. pertussis* contenant le gène codant la toxine intégrée dans le chromosome et appelée BPRA(pPT2), ou BPDS1, dans laquelle le résidu cys-41 de S1 a été délété. (Antoine, R. et al. 1990. Infect. Immun. 58:1518-1526). La sous-unité S1 est indétectable dans le surnageant de culture de cette souche, mais l'oligomère B, composant qui permet la fixation aux cellules cibles, peut être assemblé et sécrété. La capacité de la souche BPDS1 à coloniser le tractus respiratoire des souris ainsi que l'infiltration cellulaire observée au niveau pulmonaire 14 jours après administration est identique à celui de la souche BPRA. La cinétique de la réponse sérique anti-FHA après administration de la souche BPDS1 par voie intranasale (5 x 10⁶ bactéries/50 µl PBS) a été analysée sur 2 mois. Le taux observé d'anticorps IgG anti-FHA des souris BPDS1 est similaire à celui des souris BPRA et supérieur à celui détecté dans le sérum des souris BPSM (figure 6).

Ces résultats indiquent que l'activité enzymatique de PTX est impliquée dans la réduction de la réponse sérique contre l'adhésine majeure de *B. pertussis,* la FHA.

### EXEMPLE 5:

### Construction chromosomique d'une souche recombinante déficiente dans la production de toxine de B. pertussis (BPNX) exprimant une protéine Sm28GST modifiée de S. mansoni.

Une souche recombinante atténuée de *B. pertussis* a été construite afin de vérifier la corrélation entre la délétion chromosomique du gène codant pour PTX et l'augmentation de la réponse anticorps sérique dirigée contre un antigène hétérologue fusionnée à la FHA après administration intranasale de cette souche recombinante atténuée. L'antigène hétérologue modèle est un glutathion S-transférase de 28 kDa de *Schistosoma mansoni* (Sm28GST) (Balloul, J.M. et al. 1987. Nature (London). 326:149-153).

Pour éliminer le gène de PTX du génome de la souche virulente BPGR60 (Sm^{R}, Gens, Nal^{R}) de *B. pertussis,* exprimant une Sm28GST modifiée de *S*. *mansoni* en fusion avec la FHA (Renault-Mongénie, G. et al. 1996. Proc. Natl. Acad. Sci. USA. 93:7944-7949), celle-ci est croisée sur un milieu solide BG avec la souche mobilisant *E*. *coli:* S17-1 (pRIT13295) (Sm^{s}; Gen^{R}, Nal^{s}), le plasmide pRIT13295 possédant les régions flanquantes 5' et 3' du gène PTX (Antoine, Locht 1990 Infect. Immun. 58:1518-1526). Les événements de double recombinaison homologue ont lieu au niveau des régions flanquantes de ce gène. Après conjugaison, les bactéries ayant subi les deux événements de recombinaison sont sélectionnées sur des milieux sélectifs comme décrit précédemment (Antoine, et Locht, 1990. Infect. Immun. 58:1518-1526).

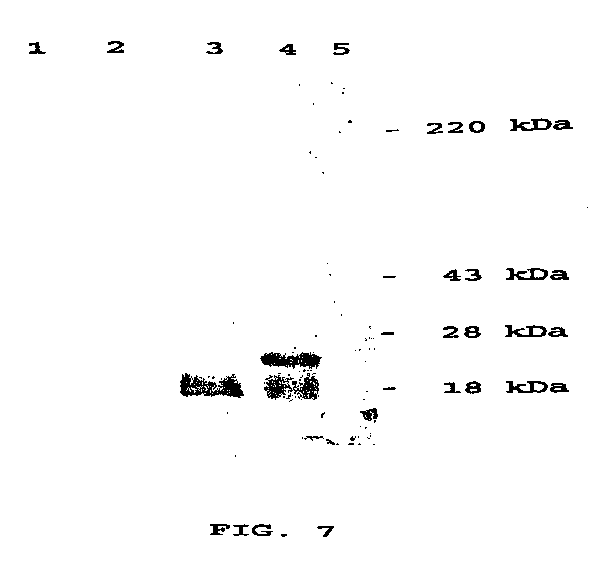

La conservation de l'antigénicité de la protéine de fusion FHA-Sm28GST et la perte de la capacité de la souche recombinante atténuée BPNX à exprimer la PTX sont vérifiées par Western-blot après séparation de la fraction protéique issue du surnageant de culture et celle associée aux cellules par SDS-PAGE sur un gel à 10%. La reconnaissance de la protéine de fusion est réalisée en utilisant des anticorps polyclonaux de rats dirigés contre la FHA et des anticorps polyclonaux de rats dirigés contre le peptide 190-211 de la Sm28GST. La toxine est identifiée dans les surnageants de culture des souches BPSM et BPGR60 à l'aide d'un anticorps monoclonal des souris 1B7 dirigée contre S1 ( Sato et al, Infect. Immun., 46, 422-428, 1984) alors qu'elle n'est pas détectée dans les surnageants des souches BPRA et BPNX (figure 7).

La souche BPNX a été déposée le 8 Octobre 1996 auprès de la CNCM sous le n° I-1770.

### EXEMPLE 6:

### Immunogénicité de la souche recombinante BPNX

La colonisation de la souche recombinante BPNX chez la souris OF1 a été vérifiée (comme cela a été décrit pour la souche BPRA dans l'exemple 1) et montre une cinétique de colonisation des poumons identique à celle de la souche sauvage de *B. pertussis.*

L'intensité de la réponse sérique IgG anti-Sm28GST obtenue chez des souris ayant reçu 5×10⁶ bactéries/50µl par voie intranasale de la souche BPNX est comparée à celle obtenue après administration de la souche BPGR60. Une restimulation des souris est effectuée, 2 mois après l'immunisation avec les souches recombinantes de *B. pertussis,* par administration intranasale de 20 µg d'antigène recombinant (rSm28GST produite chez *E*. *coli)* dans du PBS. La cinétique de la réponse immune sérique est effectuée sur des groupes de 3 à 5 souris pour chaque temps.

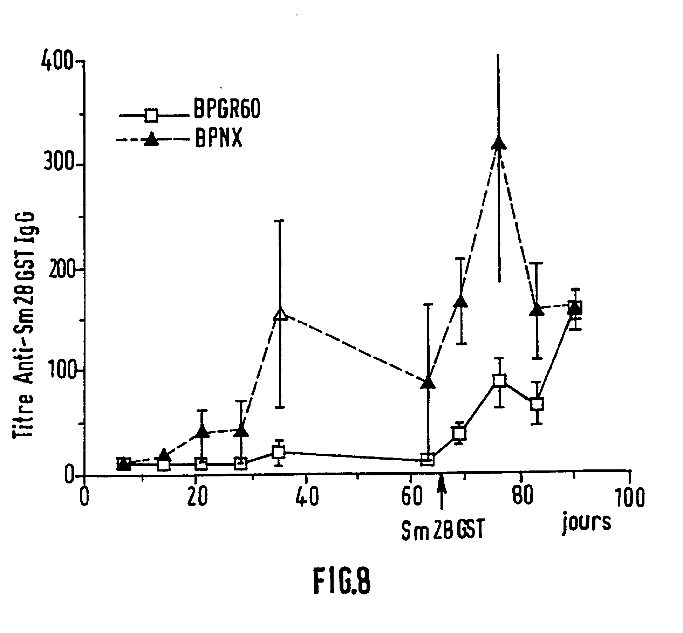

Alors qu'aucune réponse sérique spécifique de la Sm28GST n'est obtenue après administration nasale de la souche recombinante virulente BPGR60, des IgG anti-Sm28GST sont détectés dès le 14ième jour suivant l'administration de la souche recombinante atténuée BPNX et leur production augmente de manière continue jusqu'à 35 jours après administration (figure 8). Deux mois après l'immunisation, une légère diminution de ce taux d'anticorps sérique anti-Sm28GSt est observée chez les souris BPNX alors que cette réponse n'est toujours pas détectée chez les souris BPGR60. Dans les semaines qui suivent le rappel avec la rSm28GST, une production d'anticorps anti-Sm28GST est mise en évidence chez les souris BPGR60, mais elle reste plus faible que celle obtenue chez les souris BPNX.

Ces résultats montrent donc une corrélation entre la délétion chromosomique du gène codant pour PTX et l'augmentation de la réponse anticorps sérique dirigée contre un antigène hétérologue fusionné à la FHA. A quantités de bactéries similaires, une seule administration par voie intranasale d'une souche recombinante atténuée de *B. pertussis* permet donc d'induire une réponse sérique spécifique de l'antigène hétérologue fusionné à la FHA, alors qu'un rappel était nécessaire avec la souche recombinante virulente.

### EXEMPLE 7:

### Fabrication de liposomes contenant un antigène d'intérêt et présentant en surface la FHA.

L'antigène utilisé est l'enzyme glutathion S-transférase de S. *mansoni,* protéine de 28 kD, qui induit une immunité protectrice chez le mammifère, en réduisant le nombre de vers adultes et en diminuant la fécondité de ces vers. Le modèle de la Sm28GST a été utilisé comme antigène d'intérêt pour l'amélioration de ces liposomes.

Le principe est de fixer la FHA, adhésine majeure de *B. pertussis* qui possède au moins trois activités de fixation à plusieurs types cellulaires, à la surface des liposomes pour que ceux-ci adhèrent aux muqueuses par cet intermédiaire. Les résultats obtenus montrent que la présence de la FHA à la surface de liposomes contenant de la Sm28GST induit un effet dose réponse sur la réponse sérique spécifique de la Sm28GST. Cet effet serait dû au fait que la présence de cette adhésine de *B. pertussis à* la surface des liposomes entraîne une augmentation du nombre de particules atteignant les sites inducteurs. D'autre part, une homogénéisation de la réponse sérique spécifique est observée après administration de ces liposomes Sm28GST/FHA.

Des liposomes DPPC/DMPG (dipalmitoylphosphatidylcholine: dimiristoylphosphatidylglycérol) dans un rapport 9/1 formés par hydratation de préparations sèches (Phillips; N. et al. 1996. Vaccine. 14:898-904) ont été utilisés. La FHA a été purifiée à partir de surnageants de culture de *B. pertussis* (souche BPRA) par passage sur une colonne d'héparine. On a vérifié que les propriétés observées n'étaient pas dues à la présence d'endotoxines dans cette préparation. Des souris OF1 (outbred) dont l'hétérogénéité génétique est la plus représentative de ce que l'on peut observer dans une population humaine ont été utilisées.

L'administration de liposomes DPPC/DMPG contenant de la Sm28GST à des souris OF1 a permis d'obtenir une réponse immune sérique et sécrétoire spécifique de la Sm28GST.

Afin d'observer un effet d'amélioration de la réponse immune dû à l'utilisation de FHA à la surface des liposomes, les conditions sub-limites d'obtention d'une réponse immune avec des liposomes Sm28GST ont été déterminées. L'instillation nasale à 15 jours d'intervalle de liposomes fabriqués avec différentes doses de Sm28GST (5 mg/ml; 1 mg/ml; 0,2 mg/ml) pour une quantité de 2µM de phospholipides par animal et par injection, a montré que la quantité de 1 mg/ml, donnait une réponse immune faible mais décelable.

Les conditions de fabrication des liposomes qui permettent d'obtenir la présence de FHA à la surface ont aussi été déterminées. La FHA possède des séquences hydrophobes qui devraient lui permettre de s'insérer facilement dans les liposomes. Cependant, l'ajout de FHA à des liposomes Sm28GST déjà fabriqués n'a pas donné de résultats satisfaisants. Par contre, lorsque la FHA a été ajoutée à la solution de Sm28GST pour la réhydratation du film lipidique et la fabrication de liposomes, a permis d'obtenir des liposomes mixtes. La présence de FHA et de Sm28GST à la surface du liposome a été visualisée en utilisant un anticorps polyclonal contre la FHA, ou un anticorps polyclonal contre la Sm28GST.

### EXEMPLE 8:

### Augmentation de la réponse immune en fonction de la quantité de FHA.

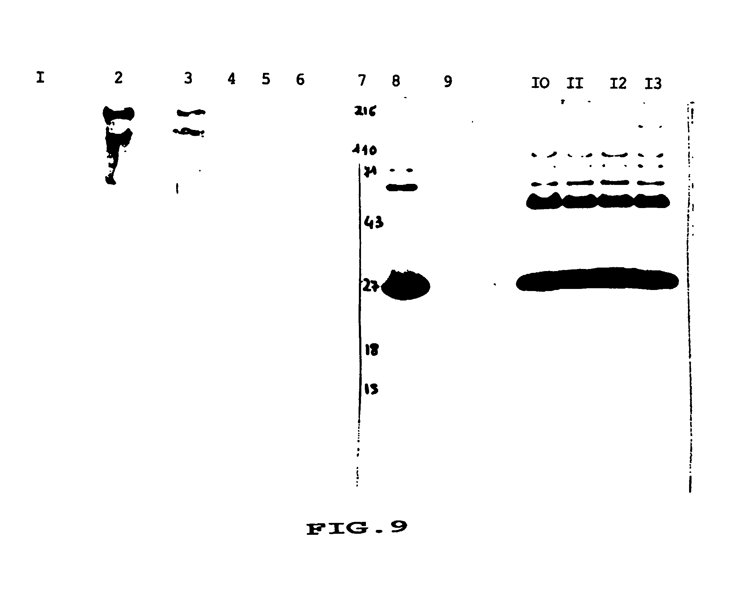

Des souris OF1 ont été immunisées par deux instillations à 15 jours d'intervalle (5 groupes contenant une quinzaine d'animaux chacun). Un groupe contrôle a été immunisé avec du PBS, un groupe a été immunisé avec des liposomes fabriqués avec 1 mg/ml de Sm28GST. Les trois autres groupes comprenaient des liposomes fabriqués avec 1 mg/ml de Sm28GST et respectivement 0.6 µg/ml; 6 µg/ml; 60 µg/ml de FHA. On a vérifié par Western-blot après séparation de la préparation liposomale par SDS-PAGE en utilisant un gel 12% que la quantité de Sm28GST incorporée dans les liposomes était identique dans les limites de résolution de la technique, en absence ou quelle que soit la quantité de FHA présente dans la solution de départ. La reconnaissance des protéines est réalisée en utilisant des anticorps polyclonaux de rats dirigés contre la FHA et des anticorps polyclonaux de rats dirigés contre le peptide 190-211 de la Sm28GST (figure 9).

Les souris ont été saignées 2 semaines après la dernière injection. L'analyse des pools de sérum a montré une augmentation de la réponse anti-Sm28GST IgG(H+L) directement dépendante de la quantité de FHA utilisée (figure 10). La réponse anti-FHA IgG (H+L) n'a été détectée que dans le groupe comportant la plus forte dose de FHA (figure 10).

L'analyse des sérums individuels a montré une homogénéisation de la réponse dans le groupe comportant une forte dose de FHA (tableau ). L'analyse des isotypes obtenus a mis en évidence une réponse mixte: (présence d'IgG1, d'IgG2a et d'IgG2b). Les courbes obtenues ont le même profil que la courbe IgG(H+L) à la fois pour la Sm28GST et pour la FHA. Une réponse mixte était aussi obtenue pour des liposomes contenant seulement la Sm28GST. Aucun effet de polarisation de la réponse n'a donc été observé.

**TABLEAU**

| Analyse de la réponse anti-Sm28GST IgG(H+L) des sérums individuels à J27 | | |
|---|---|---|
| type de liposomes | souris positives Sm28GST | souris positives FHA |
| A:Sm28GST/FHA(60) | 16/16 | 16/16 |
| B:Sm28GST/FHA(6) | 10/16 | 1/16 |
| C:Sm28GST/FHA(0,6) | 5/15 | 0/15 |
| D:Sm28GST | 5/15 | 0/15 |
| E: PBS | témoin | témoin |

## Revendications

1. Souche de *Bordetella* pertussis déficiente dans la production de toxine PTX, par élimination, délétion partielle ou mutation du gène de la toxine PTX et exprimant et exposant à sa surface une protéine hybride comprenant au moins une partie de l'hémagglutinine filamenteuse (FHA) et au moins une partie d'une protéine hétérologue vis-à-vis de la FHA.

2. Souche selon la revendication 1, **caractérisée en ce que** la protéine hétérologue comprend au moins un épitope d'une protéine susceptible d'être exprimée lors d'affections des voies respiratoires supérieures ou inférieures.

3. Souche selon la revendication 1 **caractérisée en ce que** la protéine hétérologue comprend au moins un épitope d'une protéine susceptible d'être exprimée par des pathogènes lors d'infections mucosales.

4. Souche selon la revendication 1 **caractérisée en ce que** la protéine hétérologue comprend au moins un épitope d'une protéine susceptible d'être exprimée par des pathogènes lors d'infections systémiques.

5. Souche selon la revendication 1 **caractérisée en ce que** ladite protéine hybride comprend une partie de la protéine FHA et une partie de la protéine Sm28GST de *Schistosoma mansoni.*

6. Souche BPNX selon l'une des revendications 1 et 5 **caractérisée en ce qu'**elle est celle déposée sous le N°I-1770 le 8 Octobre 1996 auprès de la Collection Nationale de Cultures de Microorganismes de l'Institut Pasteur (CNCM).

7. Procédé d'obtention d'une souche selon l'une des revendications 1 à 6 **caractérisée en ce que** le gène codant pour la toxine est éliminé de la souche virulente exprimant la protéine hybride ou au moins partiellement délété ou muté afin de produire une toxine inactive.

8. Médicament ou vaccin comprenant une souche selon l'une des revendications 1 à 6, ou obtenue selon la revendication 7.

9. Composition pharmaceutique contenant une quantité pharmacologiquement efficace d'une souche selon l'une des revendications 1 à 6 ou obtenue selon la revendications 7 et éventuellement un ou plusieurs excipients pharmaceutiquement compatibles.

10. Utilisation d'une souche selon l'une des revendications 1 à 6 ou obtenue selon la revendication 7 pour la fabrication d'un médicament ou d'un vaccin pour le traitement des affections des voies respiratoires inférieures ou supérieures.

11. Utilisation d'une souche selon l'une des revendications 1 à 6 ou obtenue selon la revendication 7 pour la fabrication d'un médicament ou d'un vaccin pour le traitement des infections mucosales.

12. Utilisation d'une souche selon l'une des revendications 1 à 6 ou obtenue selon la revendication 7 pour la fabrication d'un médicament ou d'un vaccin pour le traitement des infections systémiques.

## Claims

1. *Bordetella pertussis* strain, deficient in the production of PTX toxin by elimination, partial deletion or mutation of the PTX toxin gene, expressing and exposing at its surface an hybrid protein comprising at least one part of filamentous hemagglutinin (FHA) and at least one part of a heterologous protein to FHA.

2. Strain according to claim 1, **characterized in that** the heterologous protein comprises at least one epitope of a protein liable to be expressed upon affections of the upper or lower respiratory tract.

3. Strain according to claim 1, **characterized in that** the heterologous protein comprises at least one epitope of a protein liable to be expressed by pathogens upon mucosal infections.

4. Strain according to claim 1, **characterized in that** the heterologous protein comprises at least one epitope of a protein liable to be expressed by pathogens upon systemic infections.

5. Strain according to claim 1, **characterized in that** said hybrid protein comprises one part of the protein FHA and one part of the protein Sm28GST of *Schistosoma mansoni.*

6. Strain BPNX according to any one of claims 1 and 5, **characterized in that** it is the strain deposited under N° I-1770 on October 8, 1996, in the National Collection of Microorganism Cultures of "Institut Pasteur" (CNCM).

7. Method for obtaining a strain according to any one of claims 1 to 6, **characterized in that** the gene coding for the toxin is eliminated from the virulent strain expressing the hybrid protein or at least partially deleted or mutated so as to produce an inactive toxin.

8. Drug or vaccine comprising a strain according to any one of claims 1 to 6 or obtained according to claim 7.

9. Pharmaceutical composition containing a pharmacologically efficient quantity of a strain according to any one of claims 1 to 6 or obtained according to claim 7 and optionally one or more pharmaceutically compatible excipients.

10. Use of a strain according to any one of claims 1 to 6 or obtained according to claim 7 to produce a drug or a vaccine for treating affections of the upper or lower respiratory tract.

11. Use of a strain according to any one of claims 1 to 6 or obtained according to claim 7 to manufacture a drug or a vaccine for treating mucosal infections.

12. Use of a strain according to any one of claims 1 to 6 or obtained according to claim 7 to manufacture a drug or a vaccine for treating systemic infections.

## Patentansprüche

1. Stamm von *Bordetella pertussis,* der durch Eliminierung, partielle Deletion oder Mutation des Gens des PTX-Toxins defizient bezüglich der Produktion des PTX-Toxins ist und ein Hybridprotein, das wenigstens einen Teil des filamentösen Hämagglutinins (FHA) und wenigstens einen Teil eines gegenüber FHA heterologen Proteins umfasst, exprimiert und auf seiner Oberfläche exponiert.

2. Stamm gemäß Anspruch 1, **dadurch gekennzeichnet, dass** das heterologe Protein wenigstens ein Epitop eines Proteins umfasst, das bei Erkrankungen der oberen oder unteren Atemwege exprimiert werden kann.

3. Stamm gemäß Anspruch 1, **dadurch gekennzeichnet, dass** das heterologe Protein wenigstens ein Epitop eines Proteins umfasst, das von Erregern bei Schleimhautinfektionen exprimiert werden kann.

4. Stamm gemäß Anspruch 1, **dadurch gekennzeichnet, dass** das heterologe Protein wenigstens ein Epitop eines Proteins umfasst, das von Erregern bei systemischen Infektionen exprimiert werden kann.

5. Stamm gemäß Anspruch 1, **dadurch gekennzeichnet, dass** das Hybridprotein einen Teil des FHA-Proteins und einen Teil des Sm28GST-Proteins von *Schistosoma mansoni* umfasst.

6. BPNX-Stamm gemäß einem der Ansprüche 1 und 5, **dadurch gekennzeichnet, dass** es sich um den Stamm handelt, der am 8. Oktober 1996 bei der Collection Nationale de Cultures de Microorganismes des Institut Pasteur (CNCM) unter der Nr. I-1770 hinterlegt wurde.

7. Verfahren zur Gewinnung eines Stamms gemäß einem der Ansprüche 1 bis 6, **dadurch gekennzeichnet, dass** das Gen, das für das Toxin codiert, aus dem virulenten Stamm, der das Hybridprotein exprimiert, eliminiert ist oder wenigstens so teilweise deletiert oder mutiert ist, dass ein inaktives Toxin produziert wird.

8. Medikament oder Impfstoff, umfassend einen Stamm gemäß einem der Ansprüche 1 bis 6 oder einen Stamm, der gemäß Anspruch 7 erhalten wurde.

9. Pharmazeutische Zusammensetzung, die eine pharmakologisch wirksame Menge eines Stamms gemäß einem der Ansprüche 1 bis 6 oder eines Stamms, der gemäß Anspruch 7 erhalten wurde, sowie gegebenenfalls einen oder mehrere pharmazeutisch annehmbare Arzneimittelhilfsstoffe enthält.

10. Verwendung eines Stamms gemäß einem der Ansprüche 1 bis 6 oder eines Stamms, der gemäß Anspruch 7 erhalten wurde, zur Herstellung eines Medikaments oder Impfstoffs zur Behandlung von Erkrankungen der unteren oder oberen Atemwege.

11. Verwendung eines Stamms gemäß einem der Ansprüche 1 bis 6 oder eines Stamms, der gemäß Anspruch 7 erhalten wurde, zur Herstellung eines Medikaments oder Impfstoffs zur Behandlung von Schleimhautinfektionen.

12. Verwendung eines Stamms gemäß einem der Ansprüche 1 bis 6 oder eines Stamms, der gemäß Anspruch 7 erhalten wurde, zur Herstellung eines Medikaments oder Impfstoffs zur Behandlung von systemischen Infektionen.
